# EUROPEAN PATENT APPLICATION

(11) **EP 4 454 571 A1**
(43) Date of publication of application: **30.10.2024**
(21) Application number: 24172297.4
(22) Date of filing: 24.04.2024
(51) Int. Cl.: A61B 17/00, A61B 34/10, A61B 34/20, A61B 34/30

(54) **AUTONOMOUS NAVIGATION OF AN ENDOLUMINAL ROBOT**

(30) Priority: 26.04.2023 US 202363462228 P; 08.04.2024 US 202418629637
(71) Applicant: Covidien LP, Mansfield, MA 02048 (US)
(72) Inventor: Zhao, Jing, Lafayette, 80026 (US); Albin, Doncey M, Boulder, 80301 (US); Komp, John W., Boulder, 80301 (US)
(74) Representative: Maschio & Soames IP Ltd

(57) **Abstract**

A system and method for arriving at a biopsy or therapy site including receiving one or more images from optical sensor on a catheter, signaling a drive mechanism to articulate a distal portion of the catheter based on analysis of the one or more images, and determining that a distal portion of the catheter has reached a desired location.

## Description

### Technical Field

This disclosure relates to the field of navigation of and maintaining position of medical devices, such as biopsy or ablation tools, relative to targets.

### Description of Related Art

There are several commonly applied medical methods, such as endoscopic procedures or minimally invasive procedures, for treating various maladies affecting organs including the liver, brain, heart, lungs, gall bladder, kidneys, and bones. Often, one or more imaging modalities, such as magnetic resonance imaging (MRI), ultrasound imaging, computed tomography (CT), or fluoroscopy are employed by clinicians to identify and navigate to areas of interest within a patient and ultimately a target for biopsy or treatment. In some procedures, pre-operative scans may be utilized for target identification and intraoperative guidance. However, real-time imaging may be required to obtain a more accurate and current image of the target area. Furthermore, real-time image data displaying the current location of a medical device with respect to the target and its surroundings may be needed to navigate the medical device to the target in a safe and accurate manner (e.g., without causing damage to other organs or tissue).

For example, an endoscopic approach has proven useful in navigating to areas of interest within a patient, and particularly so for areas within luminal networks of the body such as the lungs, blood vessels, colorectal cavities, and the renal ducts. To enable the endoscopic approach, navigation systems have been developed that use previously acquired MRI data or CT image data to generate a three-dimensional (3D) rendering, model, or volume of the particular body part.

The resulting volume generated from the MRI scan or CT scan may be utilized to create a navigation plan to facilitate the advancement of a navigation catheter (or other suitable medical device) through the luminal network to an area of interest. These MRI or CT scans are typically acquired at some point prior to any navigation of the patient.

During the navigation, a locating or tracking system, such as an electromagnetic (EM) tracking system or shape sensing tracking system, may be utilized in conjunction with, for example, CT data, to facilitate guidance of the navigation catheter to the area of interest. While these systems are effective, improvements are always desired.

### SUMMARY

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a catheter system including a catheter configured for navigation within a luminal network of a patient; an optical sensor associated with a distal portion of the catheter. The system also includes a drive mechanism including a motor coupled to the catheter and configured to articulate the distal portion of the elongate catheter; and a computing device in electrical communication with the optical sensor and the drive mechanism, the computing device including a processor and a memory, the memory storing therein an application that when executed by the processor: receives images from the optical sensor, identifies a bifurcation of the luminal network in the received images, and outputs signals to the drive mechanism to articulate the distal portion of the catheter to align the distal portion of the catheter with a lumen extending from the bifurcation. Other embodiments of this aspect include corresponding computer systems, apparatus, and computer programs recorded on one or more computer storage devices, each configured to perform the actions of the methods.

Implementations may include one or more of the following features. The catheter system where the optical sensor is affixed to the distal portion of the catheter. The catheter system may include an electromagnetic sensor associated with the distal portion of the catheter and configured to detect an electromagnetic field (EM). The application when executed by the processor outputs signals to articulate the distal portion of the catheter based on the detected EM field. The application when executed by the processor receives computed tomography (CT) images, detects a location of a target within the luminal network from the CT images, generates a three-dimensional (3D) model of the luminal network, and generates a pathway within the 3D model from a location of the distal portion of the catheter and a target. The application, when executed by the processor, registers the luminal network with the 3d model. The application when executed by the processor outputs signals to the drive mechanism to autonomously articulate the distal portion of the catheter as the catheter is advanced within the luminal network. The drive mechanism is mounted on a rail, and where the application when executed by the processor advances the drive mechanism along the rail to advance the catheter into the luminal network. The catheter system may include a handle configured for manual advancement and rotation of the catheter. The catheter is configured to receive a biopsy or therapy tool. Implementations of the described techniques may include hardware, a method or process, or computer software on a computer-accessible medium.

A further aspect of the disclosure is directed to a method including receiving one or more images from an optical sensor on a catheter within a luminal network of a patient. The method also includes outputting signals to a drive mechanism coupled to the catheter to articulate a distal portion of the catheter based on the one or more images. The method also includes determining that a distal portion of the catheter has reached a location based on the one or more images. The method also includes receiving computed tomography (CT) images. The method also includes detecting a target in the CT image data. The method also includes generating a 3d model of the luminal network and the target based on the CT image data. The method also includes determining a pathway through the luminal network from the determined location of the distal portion of the catheter to the target. Other embodiments of this aspect include corresponding computer systems, apparatus, and computer programs recorded on one or more computer storage devices, each configured to perform the actions of the methods.

Implementations may include one or more of the following features. The method may include receiving electromagnetic (EM) sensor data from a sensor associated with the catheter. The method may include registering the 3D model to the luminal network based on the received EM sensor data. The method may include detecting advancement of the catheter within the luminal network based on the EM sensor data. The method may include outputting a signal to the drive mechanism to articulate the distal portion of the catheter based on the EM sensor data. The method may include detecting advancement of the catheter within the luminal network from the determined location of the distal portion of the catheter to the target and outputting a signal to the drive mechanism to articulate the distal portion of the catheter based on the detected advancement to follow the determined pathway through the luminal network. The method may include autonomously outputting a signal to the drive mechanism to articulate the distal portion of the catheter. The drive mechanism is mounted on a rail and may include outputting a signal to the drive mechanism to advance the drive mechanism along the rail to advance the catheter into the luminal network. The method may include identifying one or more locations on the target for collection of a biopsy sample. The catheter location is based on at least one property of a biopsy tool or the catheter. Implementations of the described techniques may include hardware, a method or process, or computer software on a computer-accessible medium.

### BRIEF DESCRIPTION OF THE DRAWINGS

Various aspects and embodiments of the disclosure are described hereinbelow with references to the drawings, wherein:
FIG. 1 is a schematic view of a luminal network navigation system in accordance with the disclosure;
FIG. 2 is a bronchoscopic view in accordance with the disclosure;
FIGS. 3A and 3B depict aspects of an articulation system for a catheter in accordance with the disclosure;
FIG. 4 is a flow chart of a method of local registration in accordance with the disclosure;
FIG. 5 depicts views of a robotic catheter system in accordance with the disclosure.
FIG. 6 is a user interface in accordance with the disclosure; and
FIG. 7 is a schematic view of an imaging and computing system in accordance with the disclosure.

### DETAILED DESCRIPTION

Catheters and catheter like devices, such as endoscopes, are used in a myriad of medical procedures. These flexible devices are typically used to navigate through luminal networks of the body including the vasculature, airways, and digestive systems. In accordance with the disclosure, to aide in navigating to a specific location, the distal tip of the catheter can be articulated, deflected, or rotated by a user through controls on the catheter proximal end outside the body. These manipulations allow the tip to point towards and enter branching structures. Upon arrival at the desired anatomic location a medical procedure may be performed.

A common unmet need across all flexible devices navigated within the patient is that it takes significant effort for a physician to develop proficiency in manipulating these tools. The main challenge rises from the fundamental limitation of the human brain to process high-dimensional data. Specifically, the physician often needs to mentally register the position and orientation of a 3D object (i.e., catheter) onto a 2D display (e.g., images in endoscopy, fluoroscopy, ultrasonography, etc.) while concurrently deciding how to turn handle knobs to steer the catheter towards the desired direction. On the one hand, this cognitive burden increases the risk of operator-related medical errors. On the other hand, the demanding eye-hand coordination restricts the product use to well-trained elite physicians only, preventing broad acceptance of the product in the market.

This disclosure, by incorporating machine intelligence to address this "sensor-fusion" challenge, describes systems and methods to significantly enhance the automatic navigation and aiming in catheter-based navigation procedures. Aspects of the disclosure are directed to path-planning and path-tracking algorithms that enable autonomous (e.g., robotic) navigation of a catheter to the most distal regions of the patient, with both accuracy and repeatability. These methods can be employed in a variety of products used in different minimally invasive procedures. This list includes but is not limited to bronchoscopy, transcatheter leadless pacemaker placement, transcatheter aortic valve replacement as well as others.

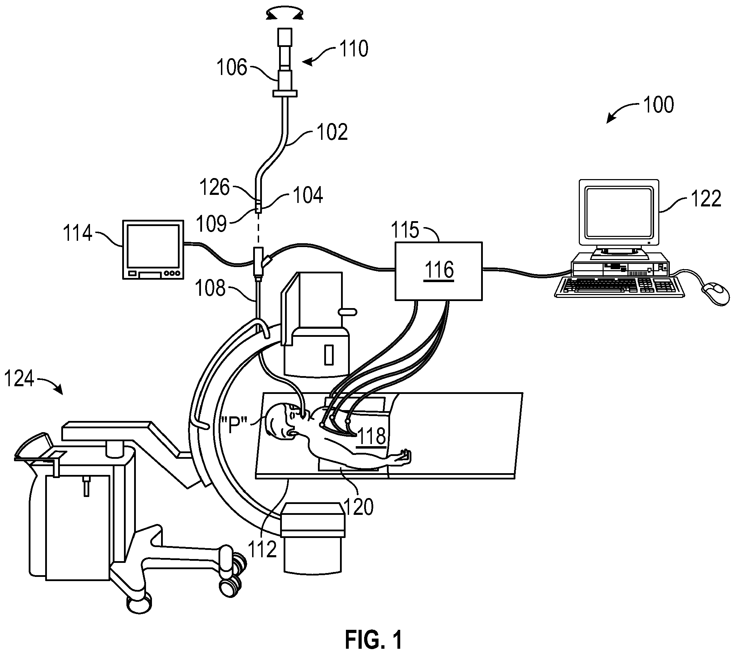

FIG. 1 is a perspective view of an exemplary system for facilitating navigation of a medical device (e.g., a catheter) to a soft tissue target via airways of the lungs. As shown in FIG. 1, catheter 102 is part of a catheter guide assembly 106. In one embodiment, catheter 102 is inserted into a bronchoscope 108 for access to a luminal network of the patient P. Specifically, catheter 102 of catheter guide assembly 106 may be inserted into a working channel of bronchoscope 108 for navigation through a patient's luminal network. The catheter 102 may itself include imaging capabilities via an integrated camera or optics component 109 and a separate bronchoscope 108 is not strictly required. A locatable guide (LG) 110 (a second catheter), including a sensor 104 may be inserted into catheter 102 and locked into position such that sensor 104 extends a desired distance beyond the distal tip of catheter 102. The position and orientation of sensor 104 relative to a reference coordinate system, and thus the distal portion of catheter 102, within an electromagnetic field can be derived. Catheter guide assemblies 106 are currently marketed and sold by Medtronic PLC under the brand names SUPERDIMENSION^{®} Procedure Kits, or EDGE^{™} Procedure Kits, and are contemplated as useable with the disclosure.

System 100 generally includes an operating table 112 configured to support a patient P. Monitoring equipment is coupled to bronchoscope 108 or catheter 102 (e.g., a video display 114, for displaying the video images received from the video imaging system of bronchoscope 108 or the catheter 102); a locating or tracking system 115 including a locating module 116, a plurality of reference sensors 118 and a transmitter mat 120 including a plurality of incorporated markers (not shown). A computing device 122 includes software and/or hardware used to facilitate identification of a target, pathway planning to the target, navigation of a medical device to the target, and/or confirmation and/or determination of placement of catheter 102, or a suitable device therethrough, relative to the target.

Catheter guide assembly 106 can be navigated within the patient and the tracking system 115 (e.g., a six degrees-of-freedom electromagnetic tracking system, or other suitable system for determining position and orientation of a distal portion of the catheter 102) is utilized, as will be outlined in greater detail to detect a position of the sensor 104 and register the patient's lungs with a 3D model generated from a CT or MRI image scan. Tracking system 115 may be configured for use with a locatable guide 110 incorporating sensor 104. As described above, locatable guide 110 and sensor 104 are configured for insertion through catheter 102 into patient P's airways (either with or without bronchoscope 108) and are selectively lockable relative to one another via a locking mechanism.

Transmitter mat 120 is positioned beneath patient P. Transmitter mat 120 generates an electromagnetic field around at least a portion of the patient P within which the position of a plurality of reference sensors 118 and the sensor 104 can be determined with use of a tracking module 116. A second electromagnetic sensor 126 may also be incorporated into the end of the catheter 102. The second electromagnetic sensor 126 may be a five degree-of-freedom sensor or a six degree-of-freedom sensor. One or more of reference sensors 118 are attached to the chest of the patient P. Registration is generally performed to coordinate locations of the three-dimensional model and two-dimensional images from the planning phase, with the patient P's airways as observed through the bronchoscope 108 and allow for the navigation phase to be undertaken with knowledge of the location of the sensor 104.

Registration of the patient P's location on the transmitter mat 120 may be performed by moving sensor 104 through the airways of the patient P. More specifically, data pertaining to locations of sensor 104, while locatable guide 110 is moving through the airways, is recorded using transmitter mat 120, reference sensors 118, and tracking system 115. A shape resulting from this location data is compared to an interior geometry of passages of a 3D model, and a location correlation between the shape and the 3D model based on the comparison is determined, e.g., utilizing the software on computing device 122. In addition, the software identifies non-tissue space (e.g., air filled cavities) in the three-dimensional model. The software aligns, or registers, an image representing a location of sensor 104 with the three-dimensional model and/or two-dimensional images generated from the three-dimension model, which are based on the recorded location data and an assumption that locatable guide 110 remains located in non-tissue space in patient P's airways. Alternatively, a manual registration technique may be employed by navigating the bronchoscope 108 with the sensor 104 to pre-specified locations in the lungs of the patient P, and manually correlating the images from the bronchoscope to the model data of the three-dimensional model.

Though described herein with respect to EMN systems using EM sensors, the instant disclosure is not so limited and may be used in conjunction with flexible sensors such as fiber-Bragg grating sensors, inertial measurement unit (IMU), ultrasonic sensors, or without sensors. Additionally, as outlined below the methods described herein may be used in conjunction with robotic systems such that robotic actuators drive the catheter 102 or bronchoscope 108 proximate the target.

In accordance with aspects of the disclosure, the visualization of intra-body navigation of a medical device (e.g., a biopsy tool or a therapy tool), towards a target (e.g., a lesion) may be a portion of a larger workflow of a navigation system. An imaging device 124 (e.g., a fluoroscope or a CT or cone beam CT imaging device such as the O-arm surgical imaging system) capable of acquiring 2D and 3D images or video of the patient P is also included in this particular aspect of system 100. The images, sequence of images, or video captured by imaging device 124 may be stored within the imaging device 124 or transmitted to computing device 122 for storage, processing, and display. Additionally, imaging device 124 may move relative to the patient P so that images may be acquired from different angles or perspectives relative to patient P to create a sequence of images, such as a fluoroscopic video. The pose of imaging device 124 relative to patient P while capturing the images may be estimated via markers incorporated with the transmitter mat 120. The markers are positioned under patient P, between patient P and operating table 112 and between patient P and a radiation source or a sensing unit of imaging device 124. The markers incorporated with the transmitter mat 120 may be two separate elements which may be coupled in a fixed manner or alternatively may be manufactured as a single unit. Imaging device 124 may include a single imaging device or more than one imaging device.

Computing device 122 may be any suitable computing device including a processor and storage medium, wherein the processor is capable of executing instructions stored on the storage medium. Computing device 122 may further include a database configured to store patient data, CT data sets including CT images, fluoroscopic data sets including images and video, 3D reconstruction, navigation plans, and any other such data. Although not explicitly illustrated, computing device 122 may include inputs, or may otherwise be configured to receive, CT data sets, fluoroscopic images/video and other data described herein. Additionally, computing device 122 includes a display configured to display graphical user interfaces. Computing device 122 may be connected to one or more networks through which one or more databases may be accessed.

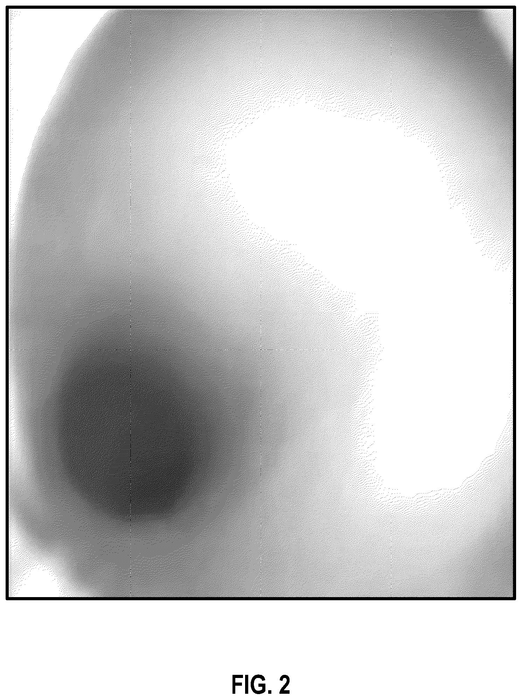

Fig. 2 depicts a typical bronchoscopic view from a catheter 102 as it is navigated within the lungs of a patient and images from optical sensor 109 are acquired. As described in greater detail below, an application running on computing device 122 analyzes these images to assess where in the patient the catheter 102 is and to make determinations on articulation and orientation of the catheter to enable further navigation.

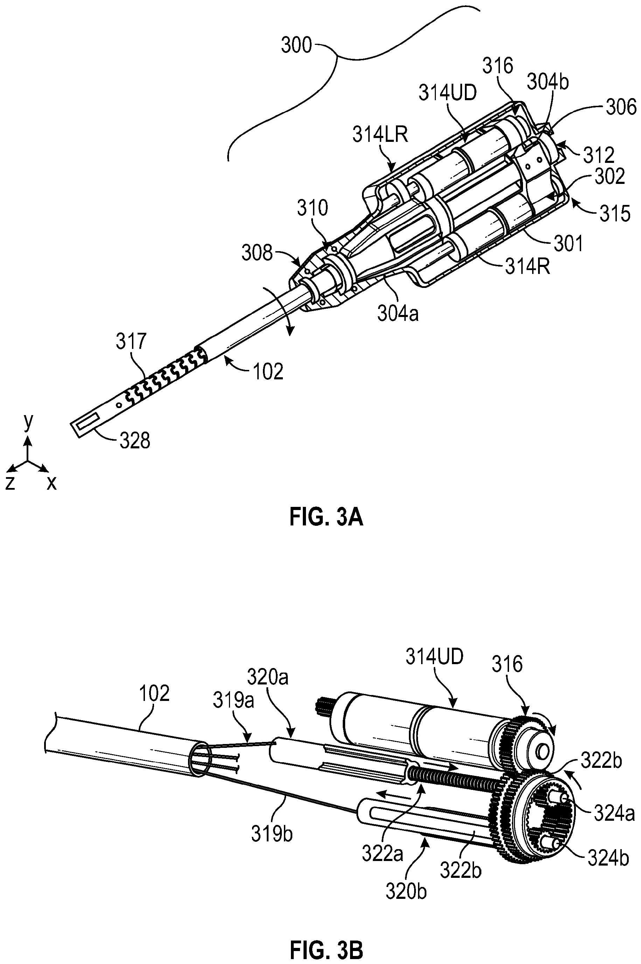

In accordance with the disclosure, the catheter 102 and its articulation and orientation relative to a target is achieved using a catheter drive mechanism 300. One example of such a drive mechanism can be seen in FIG. 3A which depicts a housing including three drive motors to manipulate a catheter extending therefrom in 5 degrees of freedom (e.g., left, right, up, down, and rotation). Other types of drive mechanisms including fewer or more degrees of freedom and other manipulation techniques may be employed without departing from the scope of the disclosure.

As noted above, FIG. 3A depicts a drive mechanism 300 housed in a body 301 and mounted on a bracket 302 which integrally connects to the body 301. The catheter 102 connects to and in one embodiment forms an integrated unit with internal casings 304a and 304b and connects to a spur gear 306. This integrated unit is, in one embodiment rotatable in relation to the body 301, such that the catheter 102, internal casings 304 a-b, and spur gear 306 can rotate about shaft axis "z". The catheter 102 and integrated internal casings 304 a-b are supported radially by bearings 308, 310, and 312. Though drive mechanism 300 is described in detail here, other drive mechanisms may be employed to enable a robot or a clinician to drive the catheter to a desired location without departing from the scope of the disclosure.

An electric motor 314R, may include an encoder for converting mechanical motion into electrical signals and providing feedback to the computing device 122. Further, the electric motor 314R (R indicates this motor if for inducing rotation of the catheter 102) may include an optional gear box for increasing or reducing the rotational speed of an attached spur gear 315 mounted on a shaft driven by the electric motor 314R. Electric motors 314LR (LR referring to left-right movement of an articulating portion 317 of the catheter 102) and 314UD (referring to up-down movement of the articulating portion 317), each motor optionally includes an encoder and a gearbox. Respective spur gears 316 and 318 drive up-down and left-right steering cables, as will be described in greater detail below. All three electric motors 314 R, LR, and UD are securely attached to the bracket 302, to prevent their rotation and enable the spur gears 315, 316, and 318 to be driven by the electric motors.

FIG. 3B depicts details of the mechanism causing articulating portion 317 of catheter 102 to articulate. Specifically, the following depicts the manner in which the up-down articulation is contemplated in one aspect of the disclosure. Such a system alone, coupled with the electric motor 314UD for driving the spur gear 316 would accomplish articulation as described above in a two-wire system. However, where a four-wire system is contemplated, a second system identical to that described immediately hereafter, can be employed to drive the left-right cables. Accordingly, for ease of understanding just one of the systems is described herein, with the understanding that one of skill in the art would readily understand how to employ a second such system in a four-wire system. Those of skill in the art will recognize that other mechanisms can be employed to enable the articulation of a distal portion of a catheter and other articulating catheters may be employed without departing from the scope of the disclosure.

To accomplish up-down articulation of the articulating portion 317 of the catheter 102, steering cables 319 a-b may be employed. The distal ends of the steering cables 319 a-b are attached to, or at, or near the distal end of the catheter 102. The proximal ends of the steering cables 319 a-b are attached to the distal tips of the posts 320 a, and 320 b. As shown in FIG. 3B, the posts 320a and 320b reciprocate longitudinally, and in opposing directions. Movement of the posts 320a causes one steering cable 319 a to lengthen and at the same time, opposing longitudinal movement of post 320 b causes cable 319 b to effectively shorten. The combined effect of the change in effective length of the steering cables 319 a-b is to cause joints a forming the articulating portion 317 of catheter 102 shaft to be compressed on the side in which the cable 319 b is shortened, and to elongate on the side in which steering cable 319 a is lengthened.

The opposing posts 320a and 320b have internal left-handed and right-handed threads, respectively, at least at their proximal ends. As shown in FIG. 3A housed within casing 304 b are two threaded shafts 322a and 322b, one is left-hand threaded and one right-hand threaded, to correspond and mate with posts 320a and 320b. The shafts 322a and 322b have distal ends which thread into the interior of posts 320a and 320b and proximal ends with spur gears 324a and 324b. The shafts 322a and 322b have freedom to rotate about their axes. The spur gears 324a and 324b engage the internal teeth of planetary gear 326. The planetary gear 326 also has external teeth which engage the teeth of spur gear 316 on the proximal end of electric motor 314UD.

To articulate the catheter in the upwards direction, a clinician may activate via an activation switch (not shown) for the electric motor 314UD causing it to rotate the spur gear 316, which in turn drives the planetary gear 326. The planetary gear 326 is connected through the internal gears 324a and 324b to the shafts 322a and 322b. The planetary gear 326 will cause the gears 324a and 324b to rotate in the same direction. The shafts 322a and 322b are threaded, and their rotation is transferred by mating threads formed on the inside of posts 320a and 320b into linear motion of the posts 320a and 320b. However, because the internal threads of post 320 a are opposite that of post 320b, one post will travel distally and one will travel proximally (i.e., in opposite directions) upon rotation of the planetary gear 326. Thus, the upper cable 319 a is pulled proximally to lift the catheter 102, while the lower cable 319 b must be relaxed. As stated above, this same system can be used to control left-right movement of the end effector, using the electric motor 314LR, its spur gear 316, a second planetary gear (not shown), and a second set of threaded shafts 322 and posts 320 and two more steering cables 319. Moreover, by acting in unison, a system employing four steering cables can approximate the movements of the human wrist by having the three electric motors 314 and their associated gearing and steering cables 319 computer controlled by the computing device 122.

In accordance with one aspect of the disclosure, as the catheter 102 is advanced into the luminal network of a patient (e.g., the airways of the lungs), an application on the computing device, can receive inputs from the camera 109, sensor 104 or sensor 126 and direct the electric motors 314 to articulate or rotate the catheter 102 to be advanced along a path to a target within the patient. In the catheter assembly 106 may be handheld by the clinician and as the clinician advances the catheter 102 into the patient, the application makes the determination of articulations of the end of the catheter 102 required to allow the catheter 102 to reach a target location. Further, the drive mechanism 300 may be incorporated into one or more robotic arms or a sled (not shown) for movement of the catheter 102 and drive mechanism 300 in the z-direction (along the longitudinal axis of the catheter 102).

The drive mechanism 300 may receive inputs from computing device 122 or another mechanism through which the surgeon specifies the desired action of the catheter 102. Where the clinician controls the movement of the catheter 102, this control may be enabled by a directional button, a joystick such as a thumb operated joystick, a toggle, a pressure sensor, a switch, a trackball, a dial, an optical sensor, and any combination thereof. The computing device responds to the user commands by sending control signals to the motors 314. The encoders of the motors 314 provide feedback to the computing device 122 about the current status of the motors 314.

In accordance with the disclosure, and as outlined in greater detail below, the drive mechanism 300 receives signals derived by the computing device 122 to drive the catheter 102 (e.g., extend and retract pull-wires) to maintain the orientation of the distal tip of the catheter 102 despite extension of a tool such as a biopsy needle or ablation catheter or movements caused by respiration and cardiac cycles.

As described in connection with FIGS. 3A and 3B, catheter 102 is operated on its proximal end through a collection of controls for rotation and distal tip deflection. In contrast, to the embodiment described in connection with FIGS. 3A and 3B, a manually advanced catheter 102 may not include the motor 314R, relying instead on manual manipulation for rotation of the catheter 102. Alternatively, the drive mechanism may include only a single wire 319, or a single pair of wires 319 a, 319 b. In such an embodiment, articulation is enabled in a single or in a pair of wires in opposite directions. One or more knobs or levers or wheels on the proximal handle control or energize the energize the respective motor 314 to enable for distal tip articulation. Rotation and advancement/extraction of the catheter 102 are controlled directly by the user's hand pushing, pulling, and rotating the catheter 102 within the patient. As described in connection with FIGS. 3A and 3B, any or all of these manual controls can be removed, and users indirectly control the catheter operation through an interface to the motors such as a joystick. Navigation may also be fully automatic with user oversight.

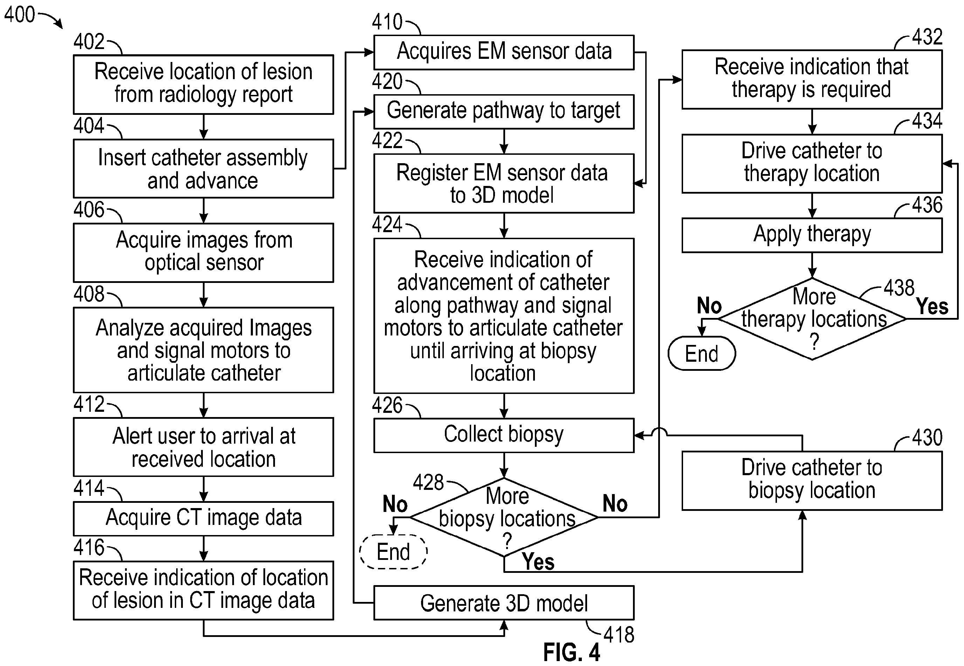

As noted elsewhere herein, the catheter assembly 106 may include an optical sensor 109 (e.g., a camera) in combination with the EM sensor 104 or EM sensor 126. A method 400 for autonomously or semi-autonomously navigating a catheter 102 employing a drive mechanism 300 to a location within a patient in accordance with the disclosure is set forth in Fig. 4. In accordance with method 400, a radiology report is received by a clinician indicating that the patient has a lesion, mass, or tumor at a location within the patient. For this example, the lesion is in a lobe of the lungs such as the right upper lobe (RUL). Once such a report is received, an examination is scheduled, and the patient is placed on the operating table 112. At step 402, the location of the lesion is entered into an application on the computing device 122. This may be as broad as RUL, or it may be more specific to identify 3^{rd} 4^{th}, or 5^{th}, etc. bifurcation in the RUL. At step 404 a catheter assembly 106 is inserted into the patient's mouth and advanced into the airways of the patient. The advancement may be manual or robotically achieved. As the catheter assembly 106 is advanced, the optical sensor 109 acquires images and identifies bifurcations of the airways at step 406. The application on the computing device 122 analyzes the images at step 408 and sends signals to the motors 314 to articulate the distal portion of the catheter 102 such that as the catheter 106 is further advanced (either manually or robotically), the shape and orientation of the distal portion of the catheter 102 is altered to align with an appropriate airway at each bifurcation to enable navigation to the desired location within the lungs of the patient (e.g., RUL).

The layout of the central airways is relatively consistent between patients despite there being distance variations between patients. Further, clinicians such as pulmonologists are well versed in the anatomy and experienced with navigating particularly the central airways of the lungs relying on visualized cues. As a result, with the input of the identity of the lobe to be reached (step 402), an application on the computing device 122 is configured to analyze the images captured by the optical sensor 109, can detect the bifurcations. With knowledge of the general anatomy of patients and an identified location to achieve, the application can autonomously direct the motors 314 to adjust the tension on the pull wires 319. Further, if additional details are entered at step 402 as to the location of the lesion (e.g., 4^{th} bifurcation RUL), the application is capable of counting the number of bifurcations as the bifurcations are observed by the optical sensor 109 and the distal portion of the catheter 102 is articulated and shaped to achieve this navigation to this location as the catheter 106 is advanced within the lungs of the patient. Similarly, the application may determine that the images acquired by the optical sensor 109 are no longer useful in assessing the anatomy of the patient.

During the advancement of the catheter assembly 106, the EM sensor 104 or 126 acquires magnetic field data generated by the transmitter matt 120 at step 410. This magnetic field data is continually acquired and may be employed in registration steps described herein below.

At step 412, once arriving at the input location from step 404, or once the optical sensor 109 is no longer capable of resolving useful images for analysis by the application on the computing device 122 an alert may be provided on a user interface on the computing device indicating that additional imaging is required. At step 414 using the imaging device 124 a CT image data set is acquired. The CT image data set may be focused on just the anatomy in question (e.g., RUL) or may be of the entire right lung, or both lungs. One benefit of focusing the CT image data set is to reduce the radiation exposure of the patient. At step 416 the target (e.g., a tumor or lesion) is identified in the CT image data set. At step 418 a 3D model of the patient's lungs, or a selected portion thereof, is generated. The generated 3D model includes a representation of the catheter 102 within the 3D model. As will be appreciated, with the catheter 102 having been navigated into the patient, at least a distal portion of the catheter 102 is captured within the CT image data and can be identified by the application running on the computing device 122 in the CT image data using a variety of image processing techniques including for example segmentation and others. At step 420 a pathway from the location of the distal portion of the catheter 102 to the target is generated. This pathway takes into account the articulation capabilities of the catheter 102 and the location of the target as well as the existence of major blood vessels to be avoided, the proximity of the parenchyma, and other physical features of the patient's lungs. Thus, the pathway may not lead directly to the tumor or lesion, where the direct pathway requires articulation that the catheter 102 and the drive mechanism 300 cannot achieve, and alternate path is identified by the application on the computing device, through the 3D model to achieve access to the target tissue.

With the target and pathway identified in the 3D model utilizing all of the EM data collected by the sensor 104, 126 in navigating to the position where the CT image data is captured and the position of the distal portion of the catheter 102 having been identified in the CT image data, the 3D model is registered with the patient at step 422. As with predecessor navigation platforms, much or all of the remainder of the navigation may be conducted without the benefit of the optical sensor 109 on the catheter 102. This is particularly true as the catheter 102 approaches the periphery of the lungs where the airways are quite narrow and images are difficult to analyze. The registration of the position of the sensor 104, 126 in the patient to the 3D model enables confident further navigation of the catheter 102 to the target.

Registration at this point in the procedure has several benefits over prior systems. As an initial matter, because the CT image data is captured with the patient in the same position as the procedure and substantially at the same time there is little to no CT-to-body divergence. CT-to-body divergence is typically a factor caused by pre-procedure capture of the CT images while the patient is at full breath hold. The CT images and the 3D model derived therefrom may be captured weeks or even months prior to the actual procedure, plus the actual procedure is not undertaken at full breath bold. Accordingly, the position and orientation of the lungs during the procedure is quite different from that observed in the pre-procedure imaging. These differences become magnified towards the periphery of the lungs. However, as noted above, this challenge is substantially eliminated by the methods described herein.

As the catheter 102 is navigated further into the periphery of the lungs the EM navigation features enabled by the registration may be phased in to greater and greater control of the articulation and orientation determination made by the application on the computing device 122. Thus, while initially the application may rely solely on image data from the optical sensor 109 to make navigational decisions, as the catheter 102 navigates distally EM sensors 104, 126 take over as the primary input to the application for controlling articulation and orientation changes of the catheter 102 to arrive at the target.

In accordance with one aspect of the disclosure the CT image data captured at step 416 is acquired at mid-tidal breathing. Further, the CT image data is captured with the patient in a natural procedure positioning, rather than with arms extended as is customarily the case. These factors, along with the catheter 102 having been navigated into the patient all combine to substantially eliminate or at minimum greatly reduce any CT-to-body divergence as compared to prior methods of intraluminal navigation.

Following registration of the detected position of the catheter 102 in the patient with the 3D model, the catheter 102 can be advanced to a target location at step 424. As the catheter 102 is advanced the application on the computing device 122 sends signals to the drive mechanism 300 to adjust the articulation and orientation of a distal portion of the catheter. The advancement and articulation or orientation changes are continued until the catheter 102 is located at the target location. The target location may be at the tumor or lesion, or where the tumor or lesion is outside of the airways, a point where a tool exchange can be undertaken.

One aspect of this advancement is that as the catheter 102 approaches the target location not only are articulation and orientation changes undertaken to enable the navigation, but also to aim the opening at the distal end of the catheter 102, through which tools such as biopsy and therapy tools are advanced, such that it is aligned with the tumor or lesion. This is another departure from prior systems, wherein aiming of the catheter 102 was typically bifurcated from the navigation of the catheter 102. As such, once the catheter 102 is at the target location, the catheter 102 is ready for the tool exchange to enable biopsy.

At step 426 a biopsy sample is collected. The application running on the computing device 122 may enable the clinician to or may automatically identify portions of the tumor or lesion from which to collect biopsies. In accordance with the disclosure, the clinician may identify one or more locations on either individual images from the CT image data, or the 3D model generated therefrom. Additionally or alternatively, an application on the computing device 122 may perform image analysis of the CT image data or 3D model and identify locations for biopsy. The clinician then may accept the biopsy locations or identify alternatives as desired. Thus, the target location to which the catheter 102 is navigated may be a first of the biopsy locations. At step 428 an inquiry is made whether there are other biopsy locations and if yes then at step 430 the application on the computing device 122 sends signals to the motors 314 to adjust the articulation of the distal portion of the catheter 102 to align the opening in the catheter 102 with the next biopsy location and another biopsy sample may be acquired. This may be repeated until all biopsy locations have been biopsied. In some instances, instructions may be displayed on the computing device 122 user interface for advancement or retraction of the catheter 102 to arrive at the desired biopsy location. After all the biopsy samples are acquired the method 400 may optionally end.

With each biopsy sample acquired, rapid on-site evaluation (e.g., pathology) may be undertaken. The results of the pathology may be accessed by the application running on the computing device 122. In such instances, determination of the need for therapy and therapy application sites (e.g., locations at which the therapy is to be applied) can be made by the clinician and the pathologist at step 432. This determination may be made in conjunction with the data regarding the locations of the biopsy sites and the selected therapy type to define the one or more therapy application sites. In a further example, the number and locations of therapy applications sites may be presented to the clinician on a user interface of the computing device 122 based on differing therapy types, any one of which may be selected by clinician. Where a therapy is required, the application running on the computing device 122 may articulate or orient the distal portion of the catheter 102 to a therapy application site at step 434 and therapy applied at step 436. A determination is made at step 438 whether further therapy sites exist. If yes at 438 the method returns to step 434 and is repeated until all therapy sites have received therapy. Once all therapy sites have received therapy, the method 400 ends.

Therapy devices in accordance with the disclosure may be one or more of a microwave ablation device, radio-frequency ablation device, an ultrasound ablation device, a chemical ablation device or others without departing from the scope of the disclosure.

As will be appreciated during steps 426 and 434 the application running on the computing device 122 can assess changes in position of the EM sensor 104, 126 and signal the drive mechanism 300 to articulate or orient the distal portion of the catheter 102 to maintain alignment with the biopsy location or therapy application site. Movement of the EM sensor 104, 126 may be caused by heat beat, respiration, or other factors.

According to another aspect of the disclosure the locations of the biopsy sites and the yields of those sites may be collected and analyzed to inform future iterations of the applications running on the computing device 122 to identify biopsy sites and to identify therapy application sites.

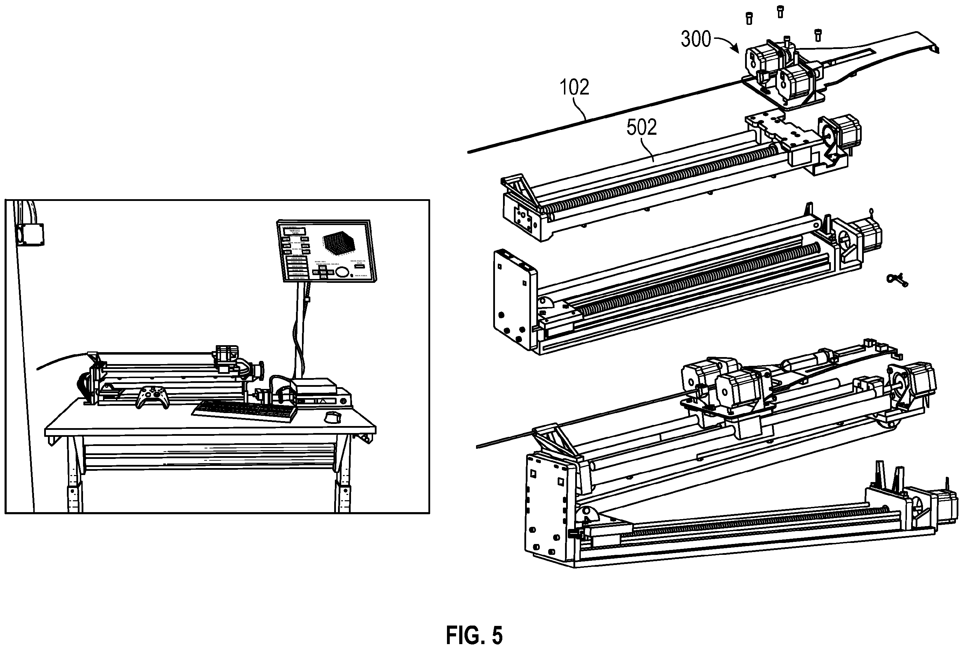

A further aspect of the disclosure is depicted in Fig. 5, where the catheter assembly 106 and the drive mechanism 300 are mounted on a rail 502. The rail 502 supports the catheter 102 and the drive mechanism 300 and the drive mechanism 300 is operatively connected to the rail to enable longitudinal or z-direction movement of the catheter 102 in accordance with the disclosure.

A further feature of the disclosure is directed to the use of markers on an exterior portion of the catheter 102. An optical sensor (not shown) but located outside of the patient (e.g., near the mouth of the patient) detects makers to determine the length of the catheter 102 inserted into the patient. This data can be received by the application running on the computing device 122 and provide another datapoint to determine the location the distal portion catheter 102 in the patient and to register the patient with the 3D model.

With respect to a planning phase, computing device 122 may optionally utilize previously acquired CT or MRI image data for generating and viewing a three-dimensional model or rendering of patient P's airways, enabling the identification of a target on the three-dimensional model (automatically, semi-automatically, or manually), and determining a pathway through patient P's airways to tissue located at and around the target. More specifically, CT images acquired from previous CT or MRI scans are processed and assembled into a three-dimensional volume, which is then utilized to generate a three-dimensional model of patient P's airways. The three-dimensional model may be displayed on a display associated with computing device 122, or in any other suitable fashion. Using computing device 122, various views of the three-dimensional model or enhanced two-dimensional images generated from the three-dimensional model are presented. The enhanced two-dimensional images may possess some three-dimensional capabilities because they are generated from three-dimensional data. The three-dimensional model may be manipulated to facilitate identification of target on the three-dimensional model or two-dimensional images, and selection of a suitable pathway through patient P's airways to access tissue located at the target can be made. Once selected, the pathway plan, three-dimensional model, and images derived therefrom, can be saved, and exported to a navigation system for use during the navigation phase(s).

As described elsewhere herein, an aspect of the system 100 is a software component including one or more applications for receiving output from the optical sensor 109 and/or EM sensors 104, 126, reviewing of CT or fluoroscopic image data, identifying one or more targets, generating a 3D model, planning a pathway to an identified target, and generating signals to (autonomously or semi-autonomously) advance, articulate, or orient (e.g., navigate) the catheter 102 within the patient to arrive at the target. These one or more applications may display a user interface on computing device 122 and can further enable registration of the patient with the acquired imaging and confirm placement of a sensor 104 126 relative to the target. The target may be tissue of interest (e.g., suspected tumors or lesions) identified by review of the CT image data during the planning phase. Following navigation, a medical device, such as a biopsy tool or therapy tool, may be inserted into catheter 102 to obtain a tissue sample from the tissue located at, or proximate to, the target or to apply therapy to the target.

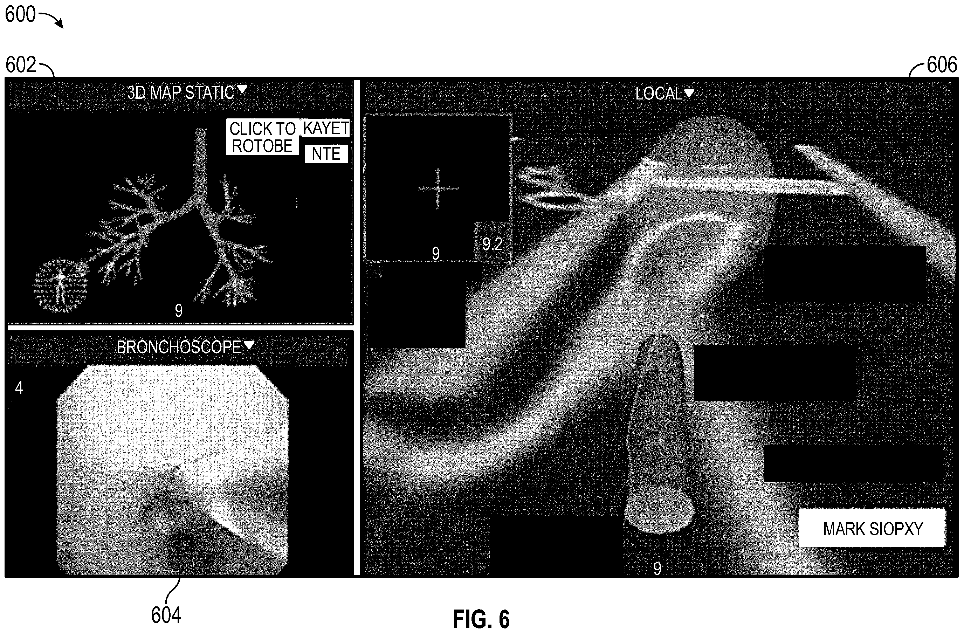

Fig. 6 depicts a user interface 600 in accordance with aspects of the disclosure. The user interface includes a variety of views, the following of which are exemplary. View 602 is an exterior 3D airway model view and indicates where in the 3D model the target is located and to what point in the model the catheter 102 has been navigated. View 604 is a live optical view and is representative of the images that the application on the computing device 122 analyzes to make articulation and orientation decisions for signaling the drive mechanism 300 to articulate orient the distal portion of the catheter 102. 3D view 606 shows an internal 3D view of the 3D model with a representation of the distal portion of the catheter 102 as it approaches the target. Other views and features of the UI may be incorporated herein without departing from the scope of the disclosure.

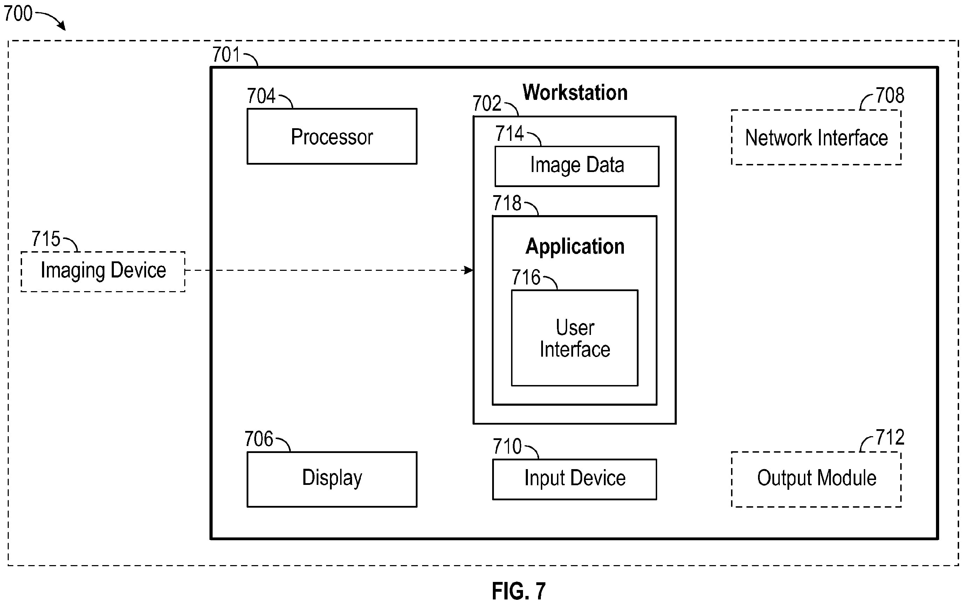

Reference is now made to FIG. 7, which is a schematic diagram of a system 700 configured for use with the methods of the disclosure including the method of FIG. 4. System 700 may include a workstation 701, and optionally an imaging device 715 (e.g., a fluoroscope, CT imaging device, or an ultrasound imaging device). In some embodiments, workstation 701 may be coupled with imaging device 715, directly or indirectly, e.g., by wireless communication. Workstation 701 may include a memory 702, a processor 704, a display 706 and an input device 710. Processor or hardware processor 704 may include one or more hardware processors. Workstation 701 may optionally include an output module 712 and a network interface 708. Memory 702 may store an application 718 and image data 77. Application 718 may include instructions executable by processor 704 for executing the methods of the disclosure including the method of FIG. 4.

Application 718 may further include a user interface 716. Image data 714 may include the CT scans, the generated fluoroscopic 3D reconstructions of the target area and/or any other fluoroscopic image data and/or the generated one or more slices of the 3D reconstruction. Processor 704 may be coupled with memory 702, display 706, input device 710, output module 712, network interface 708 and imaging device 715. Workstation 701 may be a stationary computing device, such as a personal computer, or a portable computing device such as a tablet computer. Workstation 701 may embed a plurality of computer devices.

Memory 702 may include any non-transitory computer-readable storage media for storing data and/or software including instructions that are executable by processor 704 and which control the operation of workstation 701 and, in some embodiments, may also control the operation of imaging device 715. Imaging device 715 may be used to capture a sequence of fluoroscopic images based on which the fluoroscopic 3D reconstruction is generated and to capture a live 2D fluoroscopic view according to this disclosure. In an embodiment, memory 702 may include one or more storage devices such as solid-state storage devices, e.g., flash memory chips. Alternatively, or in addition to the one or more solid-state storage devices, memory 702 may include one or more mass storage devices connected to the processor 704 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 704. That is, computer readable storage media may include non-transitory, volatile, and non-volatile, removable and non-removable media implemented in any method or technology for storage of information such as computer-readable instructions, data structures, program modules or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by workstation 1001.

Application 718 may, when executed by processor 704, cause display 706 to present user interface 716. User interface 716 may be configured to present to the user a single screen including a three-dimensional (3D) view of a 3D model of a target from the perspective of a tip of a medical device, a live two-dimensional (2D) fluoroscopic view showing the medical device, and a target mark, which corresponds to the 3D model of the target, overlaid on the live 2D fluoroscopic view. User interface 716 may be further configured to display the target mark in different colors depending on whether the medical device tip is aligned with the target in three dimensions.

Network interface 708 may be configured to connect to a network such as a local area network (LAN) consisting of a wired network and/or a wireless network, a wide area network (WAN), a wireless mobile network, a Bluetooth network, and/or the Internet. Network interface 708 may be used to connect between workstation 701 and imaging device 715. Network interface 708 may also be used to receive image data 714. Input device 710 may be any device by which a user may interact with workstation 701, such as, for example, a mouse, keyboard, foot pedal, touch screen, and/or voice interface. Output module 712 may include any connectivity port or bus, such as, for example, parallel ports, serial ports, universal serial busses (USB), or any other similar connectivity port known to those skilled in the art. From the foregoing and with reference to the various figures, those skilled in the art will appreciate that certain modifications can be made to the disclosure without departing from the scope of the disclosure.

In accordance with aspects of the disclosure, the following examples are presented.
Example 1 - A catheter system including, a catheter configured for navigation within a luminal network of a patient; an optical sensor associated with a distal portion of the catheter; a drive mechanism including a motor coupled to the catheter and configured to articulate the distal portion of the elongate catheter; and a computing device in electrical communication with the optical sensor and the drive mechanism, the computing device including a processor and a memory, the memory storing therein an application that when executed by the processor: receives images from the optical sensor, identifies a bifurcation of the luminal network in the received images, and outputs signals to the drive mechanism to articulate the distal portion of the catheter to align the distal portion of the catheter with a lumen extending from the bifurcation.
Example 2 - The catheter system of Example 1, wherein the optical sensor is affixed to the distal portion of the catheter.
Example 3 - The catheter system of Examples 1 or 2, further comprising an electromagnetic sensor associated with the distal portion of the catheter and configured to detect an electromagnetic field (EM).
Example 4 - The catheter system of any of Examples 1-3, wherein the application when executed by the processor receives computed tomography (CT) images, detects a location of a target within the luminal network from the CT images, generates a three-dimensional (3D) model of the luminal network, and generates a pathway within the 3D model from a location of the distal portion of the catheter and a target.
Example 5 - The catheter system of Example 4, wherein the application when executed by the processor registers the luminal network with the 3D model.
Example 6 - The catheter system of any of Examples 3-5, wherein the application when executed by the processor outputs signals to articulate the distal portion of the catheter based on the detected EM field.
Example 7 - The catheter system of any of Examples 1-6, wherein the application when executed by the processor outputs signals to the drive mechanism to autonomously articulate the distal portion of the catheter as the catheter is advanced within the luminal network.
Example 8 - The catheter system of Examples 1-7, wherein the drive mechanism is mounted on a rail, and wherein the application when executed by the processor advances the drive mechanism along the rail to advance the catheter into the luminal network.
Example 9 - The catheter system of Examples 1-7, further comprising a handle configured for manual advancement and rotation of the catheter.
Example 10 - The catheter system of any of Examples 1-9, wherein the catheter is configured to receive a biopsy or therapy tool.
Example 11 - A method including receiving one or more images from an optical sensor on a catheter within a luminal network of a patient; outputting signals to a drive mechanism coupled to the catheter to articulate a distal portion of the catheter based on the one or more images; determining that a distal portion of the catheter has reached a location based on the one or more images; receiving computed tomography (CT) images; detecting a target in the CT image data; generating a 3D model of the luminal network and the target based on the CT image data; and determining a pathway through the luminal network from the determined location of the distal portion of the catheter to the target.
Example 12 - The method of Example 11, further comprising receiving electromagnetic (EM) sensor data from a sensor associated with the catheter.
Example 13 - The method of Example 12, further comprising registering the 3D model to the luminal network based on the received EM sensor data.
Example 14 - The method of any of Examples 11-13, further comprising detecting advancement of the catheter within the luminal network from the determined location of the distal portion of the catheter to the target and outputting a signal to the drive mechanism to articulate the distal portion of the catheter based on the detected advancement to follow the determined pathway through the luminal network.
Example 15 - The method of any of Examples 13-14, further comprising detecting advancement of the catheter within the luminal network based on the EM sensor data.
Example 16 - The method of any of Examples 12-15, further comprising outputting a signal to the drive mechanism to articulate the distal portion of the catheter based on the EM sensor data.
Example 17 - The method of any of Examples 11-16, further comprising autonomously outputting a signal to the drive mechanism to articulate the distal portion of the catheter.
Example 18 - The method of any of Examples 11-17, wherein the drive mechanism is mounted on a rail, and further comprising outputting a signal to the drive mechanism to advance the drive mechanism along the rail to advance the catheter into the luminal network.
Example 19 - The method of any of Examples 11-18, further comprising identifying one or more locations on the target for collection of a biopsy sample.
Example 20 - The method of Example 19, further comprising determining a catheter location for each location on the target for collection of a biopsy, wherein the catheter location is based on at least one property of a biopsy tool or the catheter.

While detailed embodiments are disclosed herein, the disclosed embodiments are merely examples of the disclosure, which may be embodied in various forms and aspects. For example, embodiments of an electromagnetic navigation system, which incorporates the target overlay systems and methods, are disclosed herein; however, the target overlay systems and methods may be applied to other navigation or tracking systems or methods known to those skilled in the art. Therefore, specific structural and functional details disclosed herein are not to be interpreted as limiting, but merely as a basis for the claims and as a representative basis for teaching one skilled in the art to variously employ the disclosure in virtually any appropriately detailed structure.

The invention may be described by reference to the following numbered paragraphs:-
1. A catheter system comprising:
   a catheter configured for navigation within a luminal network of a patient;
   an optical sensor associated with a distal portion of the catheter;
   a drive mechanism including a motor coupled to the catheter and configured to articulate the distal portion of the elongate catheter; and
   a computing device in electrical communication with the optical sensor and the drive mechanism, the computing device including a processor and a memory, the memory storing therein an application that when executed by the processor:
      receives images from the optical sensor,
      identifies a bifurcation of the luminal network in the received images, and
      outputs signals to the drive mechanism to articulate the distal portion of the catheter to align the distal portion of the catheter with a lumen extending from the bifurcation.
2. The catheter system of paragraph 1, wherein the optical sensor is affixed to the distal portion of the catheter.
3. The catheter system of paragraph 1, further comprising an electromagnetic sensor associated with the distal portion of the catheter and configured to detect an electromagnetic field (EM).
4. The catheter system of paragraph 1, wherein the application when executed by the processor receives computed tomography (CT) images, detects a location of a target within the luminal network from the CT images, generates a three-dimensional (3D) model of the luminal network, and generates a pathway within the 3D model from a location of the distal portion of the catheter and a target.
5. The catheter system of paragraph 4, wherein the application when executed by the processor registers the luminal network with the 3D model.
6. The catheter system of paragraph 3, wherein the application when executed by the processor outputs signals to articulate the distal portion of the catheter based on the detected EM field.
7. The catheter system of paragraph 1, wherein the application when executed by the processor outputs signals to the drive mechanism to autonomously articulate the distal portion of the catheter as the catheter is advanced within the luminal network.
8. The catheter system of paragraph 1, wherein the drive mechanism is mounted on a rail, and wherein the application, when executed by the processor advances the drive mechanism along the rail to advance the catheter into the luminal network.
9. The catheter system of paragraph 1, further comprising a handle configured for manual advancement and rotation of the catheter.
10. The catheter system of paragraph 1, wherein the catheter is configured to receive a biopsy or therapy tool.
11. A method comprising:
   receiving one or more images from an optical sensor on a catheter within a luminal network of a patient;
   outputting signals to a drive mechanism coupled to the catheter to articulate a distal portion of the catheter based on the one or more images;
   determining that a distal portion of the catheter has reached a location based on the one or more images;
   receiving computed tomography (CT) images;
   detecting a target in the CT image data;
   generating a 3D model of the luminal network and the target based on the CT image data; and
   determining a pathway through the luminal network from the determined location of the distal portion of the catheter to the target.
12. The method of paragraph 11, further comprising receiving electromagnetic (EM) sensor data from a sensor associated with the catheter.
13. The method of paragraph 12, further comprising registering the 3D model to the luminal network based on the received EM sensor data.
14. The method of paragraph 11, further comprising detecting advancement of the catheter within the luminal network from the determined location of the distal portion of the catheter to the target and outputting a signal to the drive mechanism to articulate the distal portion of the catheter based on the detected advancement to follow the determined pathway through the luminal network.
15. The method of paragraph 13, further comprising detecting advancement of the catheter within the luminal network based on the EM sensor data.
16. The method of paragraph 12, further comprising outputting a signal to the drive mechanism to articulate the distal portion of the catheter based on the EM sensor data.
17. The method of paragraph 11, further comprising autonomously outputting a signal to the drive mechanism to articulate the distal portion of the catheter.
18. The method of paragraph 11, wherein the drive mechanism is mounted on a rail, and further comprising outputting a signal to the drive mechanism to advance the drive mechanism along the rail to advance the catheter into the luminal network.
19. The method of paragraph 11, further comprising identifying one or more locations on the target for collection of a biopsy sample.
20. The method of paragraph 19, further comprising determining a catheter location for each location on the target for collection of a biopsy, wherein the catheter location is based on at least one property of a biopsy tool or the catheter.

## Claims

1. A catheter system comprising:
a catheter configured for navigation within a luminal network of a patient;
an optical sensor associated with a distal portion of the catheter;
a drive mechanism including a motor coupled to the catheter and configured to articulate the distal portion of the elongate catheter; and
a computing device in electrical communication with the optical sensor and the drive mechanism, the computing device including a processor and a memory, the memory storing therein an application that when executed by the processor:
receives images from the optical sensor,
identifies a bifurcation of the luminal network in the received images, and
outputs signals to the drive mechanism to articulate the distal portion of the catheter to align the distal portion of the catheter with a lumen extending from the bifurcation.

2. The catheter system of claim 1, wherein the optical sensor is affixed to the distal portion of the catheter.

3. The catheter system of claim 1 or claim 2, further comprising an electromagnetic sensor associated with the distal portion of the catheter and configured to detect an electromagnetic field (EM).

4. The catheter system of any preceding claim, wherein the application when executed by the processor receives computed tomography (CT) images, detects a location of a target within the luminal network from the CT images, generates a three-dimensional (3D) model of the luminal network, and generates a pathway within the 3D model from a location of the distal portion of the catheter and a target; preferably wherein the application when executed by the processor registers the luminal network with the 3D model.

5. The catheter system of claim 3, wherein the application when executed by the processor outputs signals to articulate the distal portion of the catheter based on the detected EM field.

6. The catheter system of any preceding claim, wherein the application when executed by the processor outputs signals to the drive mechanism to autonomously articulate the distal portion of the catheter as the catheter is advanced within the luminal network; and/or wherein the drive mechanism is mounted on a rail, and wherein the application, when executed by the processor advances the drive mechanism along the rail to advance the catheter into the luminal network.

7. The catheter system of any preceding claim, further comprising a handle configured for manual advancement and rotation of the catheter; and/or wherein the catheter is configured to receive a biopsy or therapy tool.

8. A method comprising:
receiving one or more images from an optical sensor on a catheter within a luminal network of a patient;
outputting signals to a drive mechanism coupled to the catheter to articulate a distal portion of the catheter based on the one or more images;
determining that a distal portion of the catheter has reached a location based on the one or more images;
receiving computed tomography (CT) images;
detecting a target in the CT image data;
generating a 3D model of the luminal network and the target based on the CT image data; and
determining a pathway through the luminal network from the determined location of the distal portion of the catheter to the target.

9. The method of claim 8, further comprising receiving electromagnetic (EM) sensor data from a sensor associated with the catheter' preferably further comprising registering the 3D model to the luminal network based on the received EM sensor data.

10. The method of claim 8 or claim 9, further comprising detecting advancement of the catheter within the luminal network from the determined location of the distal portion of the catheter to the target and outputting a signal to the drive mechanism to articulate the distal portion of the catheter based on the detected advancement to follow the determined pathway through the luminal network; preferably further comprising detecting advancement of the catheter within the luminal network based on the EM sensor data.

11. The method of any of claims 8 to 10, further comprising outputting a signal to the drive mechanism to articulate the distal portion of the catheter based on the EM sensor data; preferably further comprising autonomously outputting a signal to the drive mechanism to articulate the distal portion of the catheter.

12. The method of any of claims 8 to 11, wherein the drive mechanism is mounted on a rail, and further comprising outputting a signal to the drive mechanism to advance the drive mechanism along the rail to advance the catheter into the luminal network.

13. The method of claim 11, further comprising identifying one or more locations on the target for collection of a biopsy sample.

14. The method of claim 13, further comprising determining a catheter location for each location on the target for collection of a biopsy, wherein the catheter location is based on at least one property of a biopsy tool or the catheter.
